# EUROPEAN PATENT APPLICATION

(11) **EP 4 785 871 A1**
(43) Date of publication of application: **05.08.2026**
(21) Application number: 24902501.6
(22) Date of filing: 19.11.2024
(51) Int. Cl.: A61B 8/00

(54) **ULTRASONIC IMAGING METHOD AND APPARATUS, COMPUTER DEVICE, MEDIUM AND COMPUTER PRODUCT**

(30) Priority: 13.12.2023 CN 202311714140
(71) Applicant: Vinno Technology (Suzhou) Co., Ltd., Jiangsu 215123 (CN)
(72) Inventor: ZHANG, Yulu, Suzhou, Jiangsu 215123 (CN); GUO, Wei, Suzhou, Jiangsu 215123 (CN); WU, Fanggang, Suzhou, Jiangsu 215123 (CN)
(74) Representative: Winter, Brandl - Partnerschaft mbB
(86) International application number: PCT/CN2024/132961
(87) International publication number: WO 2025/124079

(57) **Abstract**

The present application relates to an ultrasound imaging method, an apparatus, a computer device, a medium and a computer product. The method comprises: acquiring an ultrasound echo signal of a target region, the ultrasound echo signal is an echo signal reflected after transmitting an ultrasound wave signal of a target transmission frequency to the target region; in the target region, presetting a tracer substance, according to a magnitude relationship between the target transmission frequency and a preset resonance frequency range of the tracer substance, determining a target imaging mode corresponding to the target region; within a predetermined time period, according to the target imaging mode, performing signal acquisition on the ultrasound echo signal to obtain multiple target tracer signals, through the target imaging mode, performing localizing and tracking on each target tracer signal to obtain a target ultrasound image. By adopting the method, a high-resolution ultrasound image can be obtained, an accuracy and a precision of an ultrasound image can also be improved, and, when the method is applied to a field of medical detection, a detection cost can be reduced while a detection efficiency is improved.

## Description

The present application is based on Chinese patent application No. 202311714140.0 filed on December 13, 2023, and claims priority of the aforementioned Chinese patent application, the entire contents of which are incorporated herein by reference.

### TECHNICAL FIELD

The present application relates to the field of ultrasound imaging technology, and in particular, to an ultrasound imaging method, an apparatus, a computer device, a medium and a computer product.

### BACKGROUND

Ultrasound imaging technology is an image processing technology based on an ultrasound principle. By detecting a target object using ultrasound imaging technology, an ultrasound image showing internal structure and morphology of the target object, for example, morphology, size and position of an organ or tissue, can be obtained.

Currently, when ultrasound imaging technology is applied to a field of medical detection, during an ultrasound imaging process, an ultrasound wave is transmitted to the target object by a high-frequency ultrasound probe, then data acquisition is performed on an ultrasound wave signal reflected by the target object through the high-frequency ultrasound probe, and data analysis is performed on the collected reflected ultrasound wave signal to obtain a visible ultrasound image. However, the ultrasound image obtained by an existing ultrasound imaging method has low resolution, and when a target region of the target object is very small or in a concealed position, the accuracy of a measurement result is not high.

### SUMMARY

Based on this, it is necessary to provide an ultrasound imaging method, an apparatus, a computer device, a medium and a computer product for the above technical problems.

In a first aspect, the present application provides an ultrasound imaging method, comprising:
acquiring an ultrasound echo signal of a target region, the ultrasound echo signal being an echo signal reflected after transmitting an ultrasound wave signal of a target transmission frequency to the target region;
providing a preset tracer substance in the target region, and determining a target imaging mode corresponding to the target region according to a magnitude relationship between the target transmission frequency and a preset resonance frequency range of the tracer substance;
within a predetermined time period, performing signal acquisition on the ultrasound echo signal according to the target imaging mode to obtain multiple target tracer signals, and processing each target tracer signal through the target imaging mode to obtain a target ultrasound image.

In an embodiment, the target imaging mode comprises a two-dimensional fundamental wave imaging mode and a contrast imaging mode, and determining the target imaging mode corresponding to the target region according to the magnitude relationship between the target transmission frequency and the preset resonance frequency range of the tracer substance comprises:
when the target transmission frequency is not within the preset resonance frequency range, determining the target imaging mode as the two-dimensional fundamental wave imaging mode, wherein the two-dimensional fundamental wave imaging mode performs ultrasound imaging by acquiring a two-dimensional fundamental wave signal in the ultrasound echo signal;
when the target transmission frequency is within the preset resonance frequency range, determining the target imaging mode as the contrast imaging mode, wherein the contrast imaging mode performs ultrasound imaging by acquiring a contrast agent signal in the ultrasound echo signal.

In an embodiment, the two-dimensional fundamental wave signal comprises a tissue signal and a first tracer signal, and performing signal acquisition on the ultrasound echo signal according to the target imaging mode to obtain a target tracer signal comprises:
under the two-dimensional fundamental wave imaging mode, performing signal acquisition on the ultrasound echo signal according to the two-dimensional fundamental wave imaging mode to obtain the two-dimensional fundamental wave signal;
performing spatiotemporal filtering and frequency filtering on the two-dimensional fundamental wave signal respectively to filter out the tissue signal and obtain the first tracer signal.

In an embodiment, performing signal acquisition on the ultrasound echo signal according to the target imaging mode within a predetermined time period to obtain multiple target tracer signals comprises:
within the predetermined time period, obtaining a plurality of initial tracer signals according to movement of the tracer substance in the target region;
acquiring motion parameters of each initial tracer signal, wherein the motion parameters comprise a position parameter, a direction parameter and a velocity parameter;
when the motion parameters meet a preset parameter condition, obtaining multiple target tracer signals.

In an embodiment, before acquiring the ultrasound echo signal of the target region, the method comprises:
acquiring a first ultrasound echo signal of a target object, the first ultrasound echo signal being an echo signal reflected after transmitting an ultrasound wave signal to the target object;
obtaining a first ultrasound image according to the first ultrasound echo signal, and when a region of interest exists in the first ultrasound image, treating the region of interest as the target region.

In an embodiment, processing the target tracer signal through the target imaging mode to obtain the target ultrasound image comprises:
acquiring a first preprocessing parameter for controlling a number and an intensity of brightness in an ultrasound image, and a second preprocessing parameter for compensating movement of the target object;
performing localizing and tracking on each target tracer signal according to the first preprocessing parameter and the second preprocessing parameter to obtain an initial ultrasound image;
acquiring an image processing parameter, wherein the image processing parameter comprises image resolution, image contrast, image filtering and image enhancement;
performing image processing on the initial ultrasound image according to the image processing parameter to obtain the target ultrasound image.

In a second aspect, the present application further provides an ultrasound imaging apparatus, comprising:
a receiving module, configured to acquire an ultrasound echo signal of a target region, the ultrasound echo signal being an echo signal reflected after transmitting an ultrasound wave signal of a target transmission frequency to the target region, and determine the target transmission frequency of the ultrasound wave signal;
an imaging mode determination module, configured to provide a preset tracer substance in the target region, and determine a target imaging mode corresponding to the target region according to a magnitude relationship between the target transmission frequency and a preset resonance frequency range of the tracer substance;
an imaging module, configured to perform signal acquisition on the ultrasound echo signal according to the target imaging mode within a predetermined time period to obtain multiple target tracer signals, and perform localizing and tracking on each target tracer signal through the target imaging mode to obtain a target ultrasound image.

In a third aspect, the present application further provides a computer device, comprising a memory and a processor, the memory storing a computer program, the processor implementing the following steps when executing the computer program:
acquiring an ultrasound echo signal of a target region, the ultrasound echo signal being an echo signal reflected after transmitting an ultrasound wave signal of a target transmission frequency to the target region;
providing a preset tracer substance in the target region, and determining a target imaging mode corresponding to the target region according to a magnitude relationship between the target transmission frequency and a preset resonance frequency range of the tracer substance;
within a predetermined time period, performing signal acquisition on the ultrasound echo signal according to the target imaging mode to obtain multiple target tracer signals, and performing localizing and tracking on each target tracer signal through the target imaging mode to obtain a target ultrasound image.

In a fourth aspect, the present application further provides a computer-readable storage medium having a computer program stored thereon, the computer program implementing the following steps when executed by a processor:
acquiring an ultrasound echo signal of a target region, the ultrasound echo signal being an echo signal reflected after transmitting an ultrasound wave signal of a target transmission frequency to the target region;
providing a preset tracer substance in the target region, and determining a target imaging mode corresponding to the target region according to a magnitude relationship between the target transmission frequency and a preset resonance frequency range of the tracer substance;
within a predetermined time period, performing signal acquisition on the ultrasound echo signal according to the target imaging mode to obtain multiple target tracer signals, and performing localizing and tracking on each target tracer signal through the target imaging mode to obtain a target ultrasound image.

In a fifth aspect, the present application further provides a computer product comprising a computer program, the computer program implementing the following steps when executed by a processor:
acquiring an ultrasound echo signal of a target region, the ultrasound echo signal being an echo signal reflected after transmitting an ultrasound wave signal of a target transmission frequency to the target region;
providing a preset tracer substance in the target region, and determining a target imaging mode corresponding to the target region according to a magnitude relationship between the target transmission frequency and a preset resonance frequency range of the tracer substance;
within a predetermined time period, performing signal acquisition on the ultrasound echo signal according to the target imaging mode to obtain multiple target tracer signals, and performing localizing and tracking on each target tracer signal through the target imaging mode to obtain a target ultrasound image.

The above ultrasound imaging method, apparatus, computer device, medium and computer product, acquire an ultrasound echo signal of a target region, the ultrasound echo signal is an echo signal reflected after transmitting an ultrasound wave signal of a target transmission frequency to the target region; in the target region, a tracer substance is preset, according to a magnitude relationship between the target transmission frequency and a preset resonance frequency range of the tracer substance, a target imaging mode corresponding to the target region is determined; within a predetermined time period, according to the target imaging mode, signal acquisition is performed on the ultrasound echo signal to obtain multiple target tracer signals, through the target imaging mode, localizing and tracking is performed on each of the target tracer signals to obtain a target ultrasound image. The method determines a working mode during the ultrasound imaging process according to the target transmission frequency corresponding to the ultrasound wave signal, and can obtain a high-resolution ultrasound image at any target transmission frequency, based on the tracer substance, for a target region that is very small or a key region in a concealed position, for example, microvascular network ultrasound imaging, accuracy and precision of the ultrasound image can also be improved, meanwhile, the method can be applied to a field of medical detection, can reduce a detection cost while improving a detection efficiency.

### BRIEF DESCRIPTION OF THE DRAWINGS

To more clearly illustrate the technical solutions in the embodiments of the present application or related technologies, the accompanying drawings required for describing the embodiments or related technologies are briefly introduced below. Obviously, the drawings described below are merely some embodiments of the present application, and for those of ordinary skill in the art, other drawings may also be obtained according to these drawings without creative efforts.
FIG. 1 is an application environment diagram of an ultrasound imaging method in an embodiment;
FIG. 2 is a flow schematic diagram of an ultrasound imaging method in an embodiment;
FIG. 3 is a density schematic diagram corresponding to a tracer substance in an embodiment;
FIG. 4 is a schematic diagram of a data analysis page in an embodiment;
FIG. 5 is a flow schematic diagram of an ultrasound imaging method in another embodiment;
FIG. 6 is a structural block diagram of an ultrasound imaging device in an embodiment;
FIG. 7 is a schematic diagram of an ultrasound image display page in an embodiment;
FIG. 8 is a workflow diagram of a two-dimensional fundamental wave ultrasound imaging mode in an embodiment;
FIG. 9 is a schematic diagram of an ultrasound image mode control page in an embodiment;
FIG. 10 is a schematic diagram of a data acquisition page in an embodiment;
FIG. 11 is a schematic diagram of a data acquisition progress page in an embodiment;
FIG. 12 is a schematic diagram of a data acquisition control page in an embodiment;
FIG. 13 is a schematic diagram of a page for determining a region of interest in an embodiment;
FIG. 14 is a workflow diagram of a contrast ultrasound imaging mode in an embodiment;
FIG. 15 is a schematic diagram of a data acquisition page in another embodiment;
FIG. 16 is a structural block diagram of an ultrasound imaging apparatus in an embodiment;
FIG. 17 is an internal structure diagram of a computer device as a terminal in an embodiment;
FIG. 18 is an internal structure diagram of a computer device as a server in an embodiment.

### DETAILED DESCRIPTION

To make the objects, technical solutions and advantages of the present application more clear, the present application is described in further detail below with reference to the accompanying drawings and embodiments. It should be understood that the specific embodiments described herein are merely used to explain the present application and are not intended to limit the present application.

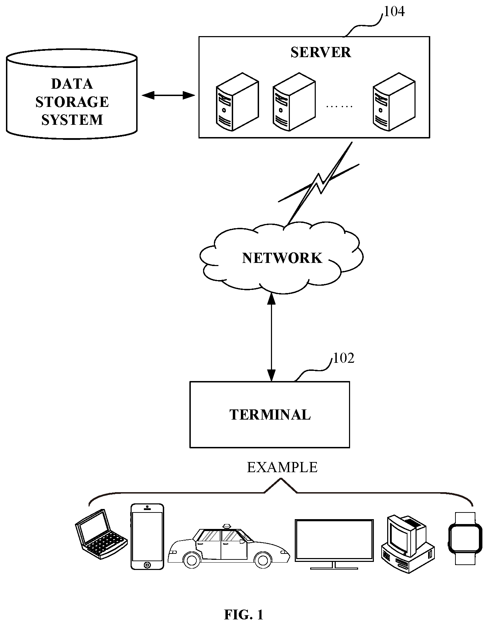

The ultrasound imaging method provided by an embodiment of the present application can be applied to an application environment as shown in FIG. 1. Wherein, a terminal 102 communicates with a server 104 through a network. A data storage system can store data that needs to be processed by the server 104. The data storage system can be integrated on the server 104, or can be placed on a cloud or other network servers. An ultrasound echo signal of a target region is acquired, the ultrasound echo signal is an echo signal reflected after transmitting an ultrasound wave signal of a target transmission frequency to the target region; in the target region, a tracer substance is preset, according to a magnitude relationship between the target transmission frequency and a preset resonance frequency range of the tracer substance, a target imaging mode corresponding to the target region is determined; within a predetermined time period, according to the target imaging mode, signal acquisition is performed on the ultrasound echo signal to obtain multiple target tracer signals, through the target imaging mode, localizing and tracking is performed on each of the target tracer signals to obtain a target ultrasound image. Wherein, the terminal 102 can be, but is not limited to, various personal computers, notebook computers, smartphones, tablet computers, Internet of Things devices, and portable wearable devices, the Internet of Things devices can be smart speakers, smart TVs, smart air conditioners, smart vehicle-mounted devices, etc. The portable wearable devices can be smart watches, smart bracelets, head-mounted devices, etc. The server 104 can be implemented by an independent server or a server cluster composed of multiple servers.

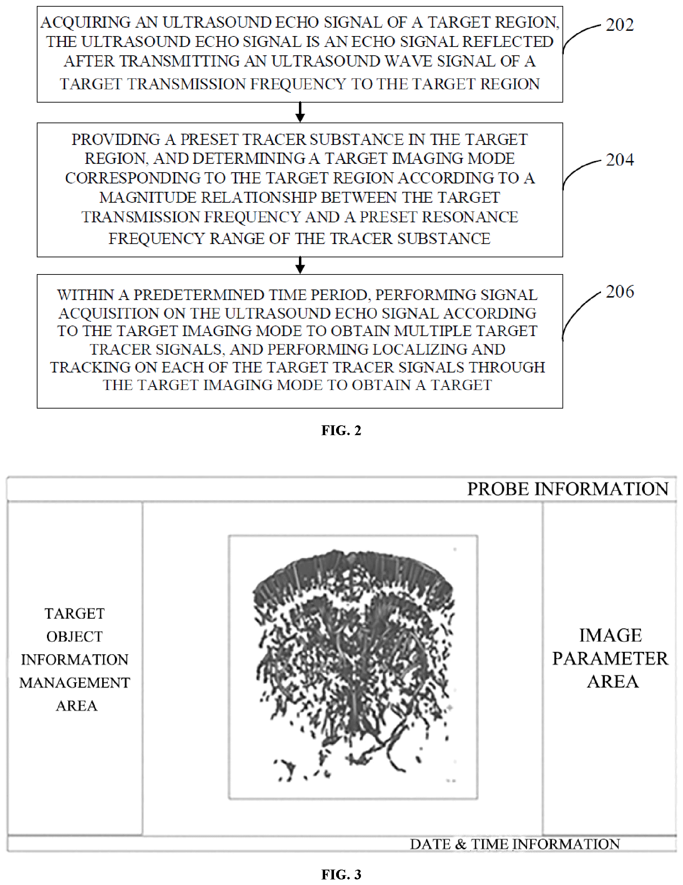

In an exemplary embodiment, as shown in FIG. 2, an ultrasound imaging method is provided, taking an application of the method to the terminal 102 in FIG. 1 as an example for description, the method comprises the following steps 202 to 206. Wherein:

Step 202, acquiring an ultrasound echo signal of a target region, the ultrasound echo signal is an echo signal reflected after transmitting an ultrasound wave signal of a target transmission frequency to the target region.

Exemplarily, taking an application of the method to an ultrasound imaging equipment as an example, in the ultrasound imaging equipment, the ultrasound imaging equipment comprises a probe, a host, an operation panel, a touch screen and a display screen. Taking a region with a pathological feature in a target object as the target region as an example, the probe in the ultrasound imaging equipment transmits an ultrasound wave signal of a target transmission frequency to the target region and receives an ultrasound echo signal. Specifically, in different tissue structures in the target region, or at an interface between tissue structures corresponding to a different type and a different density, an ultrasound wave signal has a reflection, and an echo signal reflected back is received through the probe in the ultrasound imaging equipment.

Step 204, providing a preset tracer substance in the target region, and determining a target imaging mode corresponding to the target region according to a magnitude relationship between the target transmission frequency and a preset resonance frequency range of the tracer substance.

Wherein, the tracer substance is used to enhance an ultrasound image, the tracer substance can be a tiny gas vesicle, a microbubble or other visible substance. A resonance frequency refers to a frequency at which a substance in an ultrasound field generated by an ultrasound wave signal oscillates most intensely.

Exemplarily, a preset resonance frequency range is determined according to a resonance frequency of a different tracer substance, in a microvessel of the target region, a tracer substance is preset, the tracer substance can move in the microvessel, and an ultrasound wave signal also has a reflection phenomenon at a position of the tracer substance, an echo signal reflected by the tracer substance is taken as an ultrasound echo signal corresponding to the tracer substance.

When a target transmission frequency of an ultrasound wave signal is within a preset resonance frequency range, the ultrasound wave signal has a strong resonance reaction with a tracer substance, an intensity or an energy of an ultrasound echo signal corresponding to the tracer substance is high, when the tracer substance moves in a target region, using a contrast imaging mode for signal acquisition can effectively collect the ultrasound echo signal corresponding to the tracer substance, a signal-to-noise ratio of an ultrasound image is high; when the target transmission frequency of the ultrasound wave signal is outside the preset resonance frequency range, a resonance reaction between the ultrasound wave signal and the tracer substance is weak, when the tracer substance moves in the target region, if the contrast imaging mode is used, only a weak tracer substance echo signal can be collected, the signal-to-noise ratio of the ultrasound image is low, therefore, when the target transmission frequency of the ultrasound wave signal is outside the preset resonance frequency range, a two-dimensional fundamental wave imaging mode is used for signal acquisition.

Step 206, within a predetermined time period, performing signal acquisition on the ultrasound echo signal according to the target imaging mode to obtain multiple target tracer signals, and processing each target tracer signal through the target imaging mode to obtain a target ultrasound image.

Wherein, a target tracer signal refers to an ultrasound echo signal corresponding to a tracer substance. A target ultrasound image can be a density map, a direction map and a velocity map of the tracer substance.

Exemplarily, under a contrast imaging mode, an ultrasound echo signal corresponding to a tracer substance can be directly collected to obtain a target tracer signal, according to the target tracer signal, a position of the tracer substance in a target region can be determined; under a two-dimensional fundamental wave imaging mode, signal acquisition is performed on an ultrasound echo signal to obtain a two-dimensional fundamental wave signal, wherein, the two-dimensional fundamental wave signal comprises the ultrasound echo signal corresponding to the tracer substance and an ultrasound echo signal corresponding to a tissue structure, signal processing is performed on the two-dimensional fundamental wave signal to obtain the ultrasound echo signal corresponding to the tracer substance, i.e., the target tracer signal.

Within a preset data acquisition duration, a tracer substance moves in a target region, a target tracer signal corresponding to each movement moment can be collected, localizing and tracking is performed on the target tracer signal to determine a spatial position of the tracer substance, according to a movement of the target tracer signal, a velocity magnitude and a velocity direction of the tracer substance are quantitatively calculated, and a movement trajectory of the target tracer signal corresponding to each movement moment is accumulated to obtain a density map, a velocity map and a direction map of the tracer substance in the target region, by determining a position transformation of the tracer substance in the target region, a motion video of the tracer substance can be obtained, FIG. 3 is a schematic diagram of the density map.

In the above ultrasound imaging method, under a specific ultrasound wave signal transmission frequency, an ultrasound echo signal of a target region can be acquired, the ultrasound echo signal is an echo signal reflected after transmitting an ultrasound wave signal of a target transmission frequency to the target region; in the target region, a tracer substance is preset, according to a magnitude relationship between the target transmission frequency and a preset resonance frequency range of the tracer substance, a target imaging mode corresponding to the target region is determined; within a predetermined time period, according to the target imaging mode, signal acquisition is performed on the ultrasound echo signal to obtain multiple target tracer signals, through the target imaging mode, each of the target tracer signals is processed to obtain a target ultrasound image. The method determines a working mode during an ultrasound imaging process according to a transmission frequency corresponding to an ultrasound wave signal, and can obtain a high-resolution ultrasound image at any transmission frequency, based on the tracer substance, for a target region that is very small or a key region in a concealed position, for example, microvascular network ultrasound imaging, accuracy and precision of the ultrasound image can also be improved, meanwhile, the method can be applied to a field of medical detection, can reduce a detection cost while improving a detection efficiency.

In an embodiment, a target imaging mode comprises a two-dimensional fundamental wave imaging mode and a contrast imaging mode, determining the target imaging mode corresponding to a target region according to a magnitude relationship between a target transmission frequency and a preset resonance frequency range of a tracer substance comprises: when the target transmission frequency is not within the preset resonance frequency range, determining the target imaging mode as the two-dimensional fundamental wave imaging mode, wherein, the two-dimensional fundamental wave imaging mode performs ultrasound imaging by acquiring a two-dimensional fundamental wave signal in an ultrasound echo signal; when the target transmission frequency is within the preset resonance frequency range, determining the target imaging mode as the contrast imaging mode, wherein, the contrast imaging mode performs ultrasound imaging by acquiring a contrast agent signal in the ultrasound echo signal.

Wherein, a two-dimensional fundamental wave signal refers to a fundamental wave signal propagating in two spatial dimensions, for example, on an x-axis and a y-axis, the fundamental wave signal is a component having a lowest frequency in a spectrum of a signal. A contrast agent signal refers to an ultrasound echo signal related to a tracer substance, acquired for imaging by presetting a tracer substance, for example, presetting a contrast tracer agent, in a target object during an ultrasound imaging process.

Exemplarily, when a target transmission frequency is not within a preset resonance frequency range, a two-dimensional fundamental wave imaging mode is used to perform signal acquisition on a two-dimensional fundamental wave signal in an ultrasound echo signal, in this imaging mode, the two-dimensional fundamental wave signal comprises an echo signal obtained from a phenomenon such as a reflection, a scattering, a diffraction of an ultrasound wave signal by a different tissue structure in a target region, therefore an ultrasound image comprises tissue information of each tissue structure in the target region. For example, when a tracer substance is a microbubble comprising an inert gas, there is a large acoustic impedance difference between the tracer substance and a tissue structure, an intensity of an echo signal reflected by the tissue structure from the ultrasound wave signal is high, a noise signal in the echo signal can be removed, wherein, an acoustic impedance refers to a propagation obstacle that exists when an ultrasound wave propagates between different tissue structures, the tissue structure and a tissue function can be distinguished and determined through an echo signal generated by an acoustic impedance of a different tissue structure. Further, when the tracer substance moves in the target region, for example, when the tracer substance moves with blood in a tissue vascular network, a movement of the tracer substance and a movement of other extravascular tissue structure have an obvious movement difference, therefore, an ultrasound echo signal acquired during a movement process of the tracer substance can be distinguished from an ultrasound echo signal acquired from a movement of a tissue structure, and then a first tracer signal corresponding to the tracer substance can be determined in the two-dimensional fundamental wave signal.

When the target transmission frequency is within the preset resonance frequency range, a contrast imaging mode is used, in the contrast imaging mode, an ultrasound wave signal is processed by a pulse inversion, an amplitude modulation, a contrast pulse sequence, etc., to obtain a target ultrasound wave signal sequence, the target ultrasound wave signal sequence is manifested as a different aperture and a different polarity. After the target ultrasound wave signal sequence is sent to the target region, the target ultrasound wave signal sequence is superimposed with the ultrasound echo signal, which can suppress an ultrasound echo signal corresponding to a tissue structure with a smaller movement amplitude, and can directly obtain the ultrasound echo signal corresponding to the movement of the tracer substance for ultrasound imaging, i.e., a contrast agent signal.

In this embodiment, under a different ultrasound imaging mode, a different ultrasound echo signal is acquired, based on a characteristic of the different ultrasound echo signal, an ultrasound echo signal related to a tracer substance, i.e., a tracer signal, can be well separated, since a separation effect of the tracer signal is good, a position tracking of the tracer substance can be achieved according to the tracer signal, and then a high-resolution ultrasound image of a target region can be obtained. And, if the method is applied to a field of medical detection, under a contrast imaging mode, a contrast agent signal and the tracer signal can be acquired simultaneously, according to the contrast agent signal, a detection analysis of a conventional contrast mode can be achieved, according to the tracer signal, a detection analysis of a super-resolution contrast imaging mode can be achieved, which can reduce a detection cost while improving a detection efficiency.

In an embodiment, a two-dimensional fundamental wave signal comprises a tissue signal and a first tracer signal, and performing signal acquisition on the ultrasound echo signal according to the target imaging mode to obtain a target tracer signal comprises: under a two-dimensional fundamental wave imaging mode, performing signal acquisition on the ultrasound echo signal according to the two-dimensional fundamental wave imaging mode to obtain the two-dimensional fundamental wave signal; performing a spatiotemporal filtering and a frequency filtering on the two-dimensional fundamental wave signal respectively, to filter out the tissue signal, to obtain the first tracer signal.

Wherein, a spatiotemporal filtering refers to performing a filtering operation on a gray value of each pixel in an ultrasound image, combining or calculating a pixel value around each pixel in an original ultrasound image to obtain a target ultrasound image corresponding to a new pixel value, the spatiotemporal filtering can be used for an image enhancement and an image denoising. A frequency filtering refers to performing a Fourier transform on the ultrasound image, converting the ultrasound image from a time domain to a frequency domain, then performing a filtering operation in the frequency domain, and finally converting the ultrasound image back to the time domain through an inverse Fourier transform to obtain a filtered ultrasound image, the frequency filtering can be used for an image denoising, a frequency domain enhancement and an image restoration.

Exemplarily, as described in the aforementioned embodiment, under a two-dimensional fundamental wave imaging mode, an ultrasound echo signal of a tissue structure, i.e., a tissue signal, can be acquired, and an ultrasound echo signal corresponding to a tracer substance, i.e., a first tracer signal, can also be acquired. A filtering is used to perform a filtering processing on a two-dimensional fundamental wave signal, the tissue signal in the two-dimensional fundamental wave signal can be filtered out to obtain the first tracer signal. Specifically, in a spatiotemporal filtering, a spatial-temporal filtering method based on a time difference is used, this spatial-temporal filtering method is based on a fact that a time delay of a tracer signal propagating in a target region is different from a time delay of the tissue signal in the tissue structure, by acquiring multiple frames of two-dimensional fundamental wave signals and performing a time difference on the two-dimensional fundamental wave signals, a set of time difference images is obtained. Then, through a frequency domain filtering processing, the set of time difference images is processed to separate the tissue signal and the tracer signal, for example, a high-pass filter can be used to suppress a low-frequency component of the tissue signal, while retaining a high-frequency tracer signal, to obtain the first tracer signal under the two-dimensional fundamental wave imaging mode.

In this embodiment, a spatiotemporal filtering and a frequency filtering can be adjusted according to a characteristic of a tissue signal, by filtering out an interference of the tissue signal, a first tracer signal is made more prominent, which helps to improve a contrast and a clarity of an ultrasound image. Through the filtering processing, an intensity and a visibility of the first tracer signal can also be enhanced, which helps to accurately detect and locate a tracer substance. The filtering processing can also help to improve a spatial resolution and a frequency resolution of the ultrasound image, by removing a noise, an image blur and an artifact can be reduced, providing a more accurate and detailed image information.

In an embodiment, performing signal acquisition on the ultrasound echo signal according to the target imaging mode within a predetermined time period to obtain multiple target tracer signals comprises: within the predetermined time period, obtaining a plurality of initial tracer signals according to movement of the tracer substance in the target region; acquiring a motion parameter of each initial tracer signal, wherein, the motion parameter comprises a position parameter, a direction parameter and a velocity parameter; when the motion parameter meets a preset parameter condition, obtaining multiple target tracer signals.

Wherein, an initial tracer signal is an ultrasound echo signal of a tracer substance.

Exemplarily, a data acquisition time corresponding to a target imaging mode is determined, the data acquisition time is taken as a predetermined time period, taking the target imaging mode as a two-dimensional fundamental wave imaging mode as an example, an ultrasound imaging equipment can perform multiple data acquisition works on a two-dimensional fundamental wave signal of a target region based on the two-dimensional fundamental wave imaging mode, each data acquisition work can acquire one or more two-dimensional fundamental wave signals, multiple initial tracer signals can be obtained by performing a filtering processing on each two-dimensional fundamental wave signal. The initial tracer signal comprises a data acquisition label, used to characterize a data acquisition time or a data acquisition round number corresponding to the initial tracer signal. According to the initial tracer signal, a position, a direction and a velocity of a tracer substance in the target region can be determined, based on the data acquisition label, the position, the direction and the velocity of each initial tracer signal are compared, for example, if a velocity of the tracer substance at a second moment is much greater than a velocity of the tracer substance at other moments, or the velocity of the tracer substance at the second moment is greater than a preset velocity value; or a distance of the position of the tracer substance at the second moment from a position of the tracer substance at other moments is much greater than a preset distance value; or a direction of the tracer substance at the second moment is different from a direction of the tracer substance at all other moments, the initial tracer signal corresponding to the tracer substance at the second moment can be considered as an abnormal signal, the initial tracer signal at the second moment is deleted from the initial tracer signals, to obtain multiple target tracer signals.

In this embodiment, based on a preset parameter condition, an abnormal initial tracer signal can be excluded, a noise and an artifact in an image can be reduced, thereby improving a quality and a clarity of the image. In a tracking process of an initial tracer signal, excluding the abnormal initial tracer signal can improve a tracking effect of a tracer substance, and improve a stability and a reliability of an ultrasound image.

In an embodiment, before acquiring an ultrasound echo signal of a target region, the method comprises: acquiring a first ultrasound echo signal of a target object, the first ultrasound echo signal is an echo signal reflected after transmitting an ultrasound wave signal to the target object; obtaining a first ultrasound image according to the first ultrasound echo signal, and when a region of interest exists in the first ultrasound image, treating the region of interest as the target region.

Exemplarily, an ultrasound wave signal is transmitted to a target object through an ultrasound imaging equipment, and a first ultrasound echo signal obtained after the target object reflects the ultrasound wave signal is received, according to the first ultrasound echo signal, an overall detection can be performed on the target object to obtain a first ultrasound image corresponding to the target object. Based on the first ultrasound image, a region with a pathological feature can be taken as a region of interest, and a high-resolution ultrasound imaging mode is further used for the region of interest to obtain a target ultrasound image.

In this embodiment, after a target region of a target object is determined according to a region of interest, an ultrasound imaging process of the target region is then performed, which can reduce a data analysis amount of ultrasound imaging, reduce an analysis time and an analysis cost, and improve a working efficiency of the ultrasound imaging process.

In an embodiment, processing a target tracer signal through a target imaging mode to obtain a target ultrasound image comprises: acquiring a first preprocessing parameter for controlling a number and an intensity of a brightness in an ultrasound image, and a second preprocessing parameter for compensating a movement of a target object; performing an analysis on each target tracer signal according to the first preprocessing parameter and the second preprocessing parameter to obtain an initial ultrasound image; acquiring an image processing parameter, wherein, the image processing parameter comprises an image resolution, an image contrast, an image filtering and an image enhancement; performing an image processing on the initial ultrasound image according to the image processing parameter to obtain the target ultrasound image.

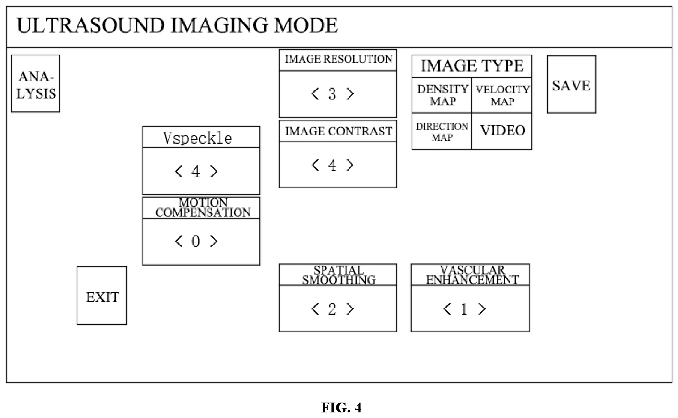

Exemplarily, as shown in FIG. 4, in an ultrasound imaging process of a target region, in a process of performing a signal analysis on a target tracer signal to obtain an ultrasound image, a first preprocessing parameter can be acquired, for example, a Vspeckle parameter corresponding to a special adaptive imaging processing technology. If the Vspeckle parameter is smaller, an information amount in the ultrasound image obtained through the signal analysis is larger, and a noise of the ultrasound image is more; if the Vspeckle parameter is larger, the information amount in the ultrasound image obtained through the signal analysis is less, and the noise of the ultrasound image is less, the ultrasound image is cleaner. A second preprocessing parameter is acquired, for example, a motion compensation parameter related to a motion compensation algorithm, the second preprocessing parameter is determined according to a movement of a tissue structure during a data acquisition process. If the motion compensation parameter is smaller, a suppression effect of the motion compensation algorithm on a tissue structure movement during a data acquisition is weaker, but a computation amount for the target tracer signal is also smaller, and an analysis duration is shorter; if the motion compensation parameter is larger, the suppression effect of the motion compensation algorithm on the tissue structure movement during the data acquisition is stronger, and the analysis duration is longer. According to an imaging requirement of the ultrasound image, the first preprocessing parameter and the second preprocessing parameter are determined, through the first preprocessing parameter and the second preprocessing parameter, a signal analysis is performed on each target tracer signal, an initial ultrasound image corresponding to the target region can be obtained. An image processing is performed on the initial ultrasound image, the image processing can be adjusting an image processing parameter such as an image resolution, a contrast, a spatial smoothing, a vascular enhancement, etc., to obtain a target ultrasound image.

Further, a measurement can be performed on a target ultrasound image, for example, when a tracer substance moves in a microvessel of a target region, a parameter such as a microvessel diameter, a spacing, a density, a complexity, a tortuosity, a perfusion index can be measured. And, to improve an accuracy of a measurement result and an accuracy of a microvascular network, a magnification processing can be performed on an initial ultrasound image, during an image magnification process, a resolution and a display detail of the initial ultrasound image are dynamically increased, to improve an accuracy precision of the measurement result.

In this embodiment, during an ultrasound imaging process, a movement of a tissue structure of a target object will cause an ultrasound image to be blurry or have an artifact, through a motion compensation technology, an image deformation caused by the movement can be corrected, improving a clarity and an accuracy of the image. After an ultrasound image is obtained, an adjustment is made to a parameter such as a brightness, a color and a filtering of the ultrasound image, which can improve a quality and a readability of the ultrasound image, and improve a signal-to-noise ratio and a clarity of the image.

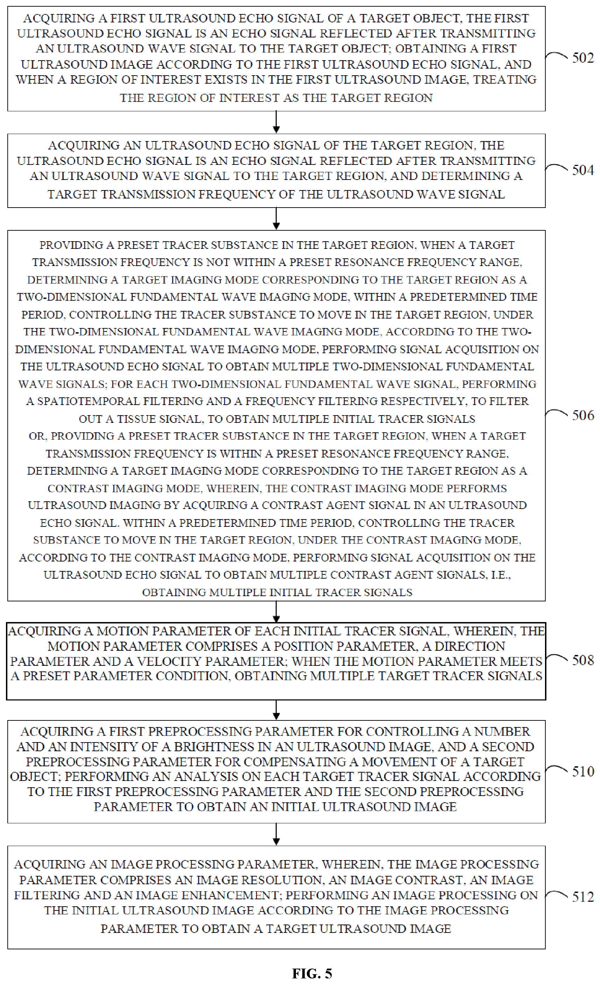

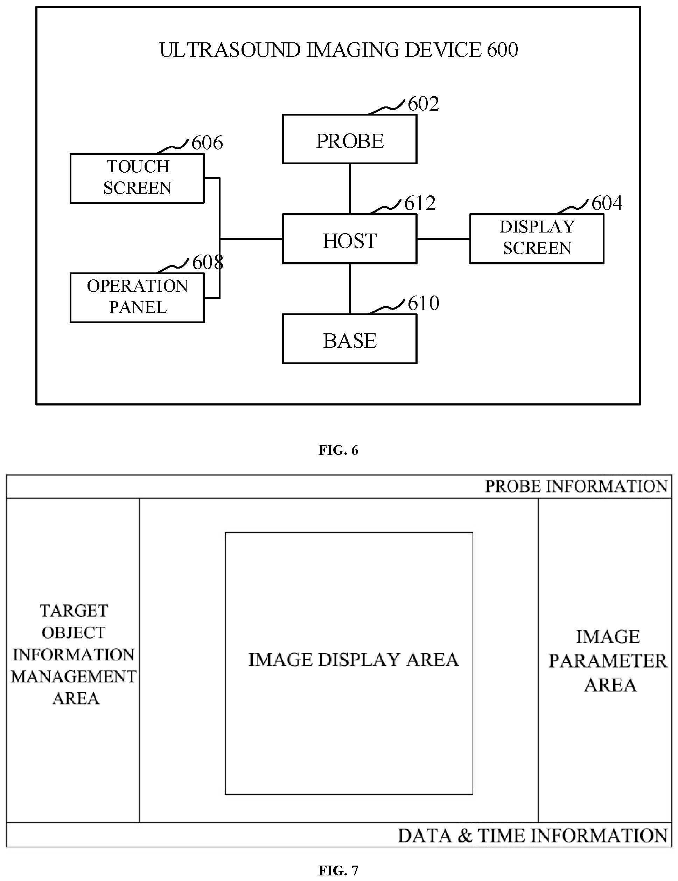

In an exemplary embodiment, as shown in FIG. 5, an ultrasound imaging method is provided, the method is applied to an ultrasound imaging device as shown in FIG. 6, the method comprises:

Step 502, acquiring a first ultrasound echo signal of a target object, the first ultrasound echo signal is an echo signal reflected after transmitting an ultrasound wave signal to the target object; obtaining a first ultrasound image according to the first ultrasound echo signal, and when a region of interest exists in the first ultrasound image, treating the region of interest as the target region.

Step 504, acquiring an ultrasound echo signal of the target region, the ultrasound echo signal is an echo signal reflected after transmitting an ultrasound wave signal to the target region, and determining a target transmission frequency of the ultrasound wave signal.

Step 506, providing a preset tracer substance in the target region, when a target transmission frequency is not within a preset resonance frequency range, determining a target imaging mode corresponding to the target region as a two-dimensional fundamental wave imaging mode, wherein, the two-dimensional fundamental wave imaging mode performs ultrasound imaging by acquiring a two-dimensional fundamental wave signal in an ultrasound echo signal. within a predetermined time period, controlling the tracer substance to move in the target region, under the two-dimensional fundamental wave imaging mode, according to the two-dimensional fundamental wave imaging mode, performing signal acquisition on the ultrasound echo signal to obtain multiple two-dimensional fundamental wave signals; for each two-dimensional fundamental wave signal, performing a spatiotemporal filtering and a frequency filtering respectively, to filter out a tissue signal, to obtain multiple initial tracer signals.

Or, providing a preset tracer substance in the target region, when a target transmission frequency is within a preset resonance frequency range, determining a target imaging mode corresponding to the target region as a contrast imaging mode, wherein, the contrast imaging mode performs ultrasound imaging by acquiring a contrast agent signal in an ultrasound echo signal. Within a predetermined time period, controlling the tracer substance to move in the target region, under the contrast imaging mode, according to the contrast imaging mode, performing signal acquisition on the ultrasound echo signal to obtain multiple contrast agent signals, i.e., obtaining multiple initial tracer signals.

Exemplarily, taking an ultrasound imaging device shown in FIG. 6 as an example, an ultrasound imaging device 600 comprises a probe 602, a display screen 604, a touch screen 606, an operation panel 608, a base 610 and a host 612, wherein, the host 612 is connected to the probe 602, the display screen 604, the touch screen 606, the operation panel 608, and the base 610 respectively, the display screen 604 is used to display a diagnostic information, as shown in FIG. 7, the display screen 604 comprises an image display area, a target object information management area and an image parameter area, the image display area is used to display an ultrasound image, the image parameter area is used to display a parameter corresponding to the ultrasound image, and the target object information management area is used to display an information of a target object; the touch screen 606 and the operation panel 608 are used to select and implement a different application or function; the host 612 comprises a hardware such as a processor, a motherboard, a hard disk, for controlling the ultrasound imaging device 600; a chassis 610 is used to fix or move the ultrasound imaging device 600, and a free movement of the ultrasound imaging device 600 can be achieved through a roller of the chassis.

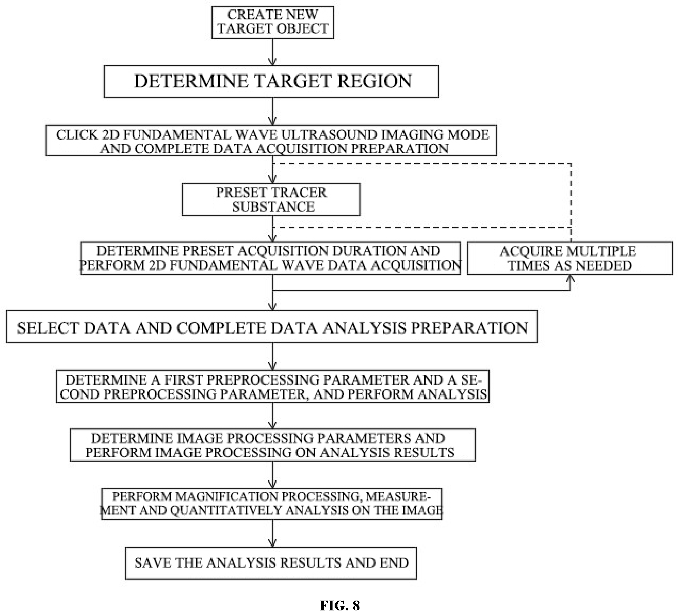

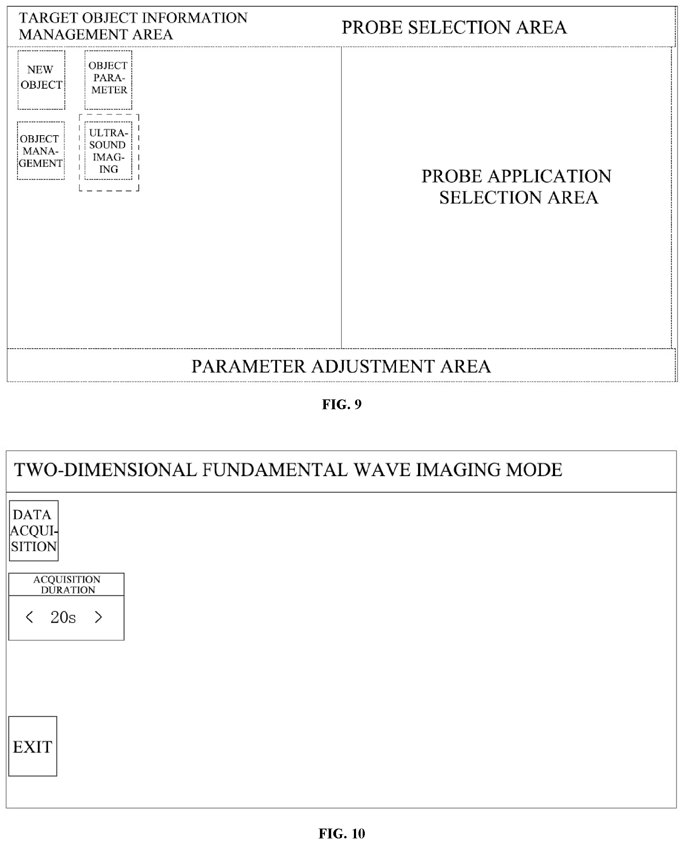

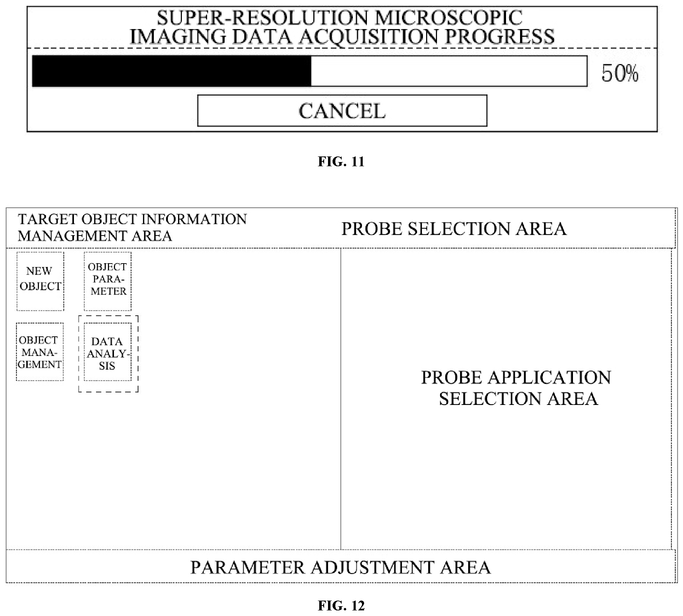

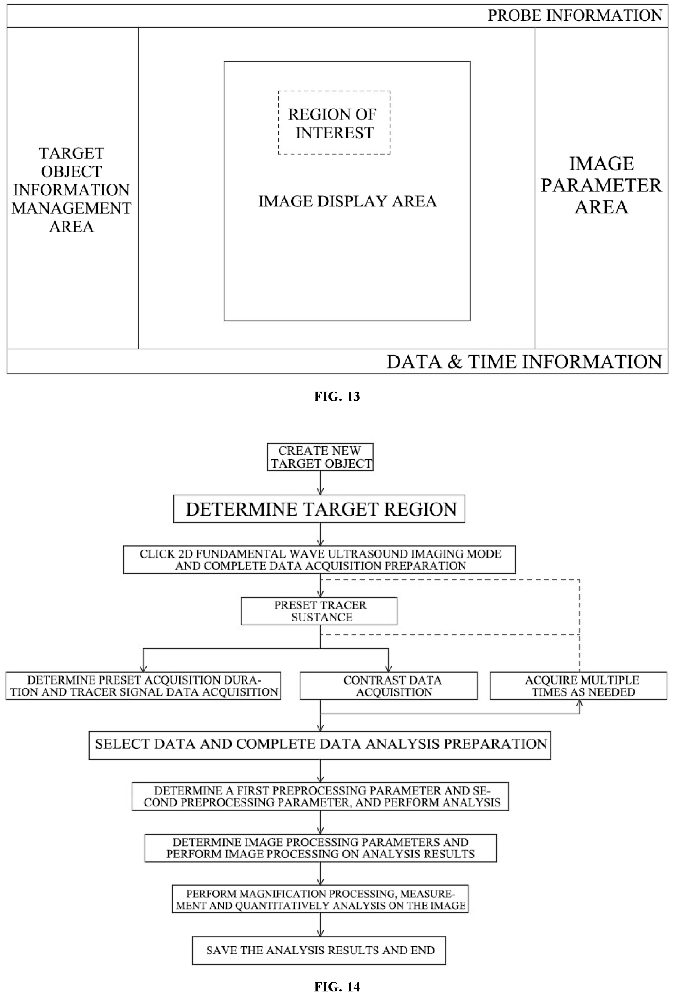

Taking a tracer substance as a contrast tracer agent as an example, the contrast tracer agent moves in a microvascular network of a target region, the probe 602 of the ultrasound imaging device 600 can adopt multiple ultrasound imaging modes such as a two-dimensional fundamental wave imaging, a color Doppler imaging, and a power Doppler imaging. If the probe 602 is a high-frequency probe, under a higher ultrasound wave transmission frequency, a resonance phenomenon amplitude of the contrast tracer agent is weak, at this time the ultrasound imaging device 600 works in a two-dimensional fundamental wave ultrasound imaging mode, FIG. 8 is a workflow diagram corresponding to the two-dimensional fundamental wave ultrasound imaging mode. In a display screen page as shown in FIG. 9, clicking an ultrasound imaging button responds to an instruction to start the two-dimensional fundamental wave ultrasound imaging mode, an ultrafast plane wave fundamental wave imaging is enabled, and a data acquisition preparation is completed. After clicking the ultrasound imaging button, a display screen page as shown in FIG. 10 is entered, on this display screen page, after a preset acquisition duration is determined, clicking a data acquisition button responds to a data acquisition instruction to perform a data acquisition on a two-dimensional fundamental wave signal. During a data acquisition process, as shown in FIG. 11, the display screen page can display a data acquisition progress in real time, when a progress bar reaches 100%, one data acquisition process is completed, a set of two-dimensional fundamental wave signal data is obtained, and at the same time, a marker image corresponding to the set of two-dimensional fundamental wave signal data can be generated in the target object information management area of the display screen 604. When the preset acquisition duration is long, a cancel button below the progress bar can be clicked to respond to a cancel data acquisition instruction, manually ending the data acquisition process to obtain multiple sets of two-dimensional fundamental wave signal data, clicking the generated marker image, the display screen page can play back the marker image. In the display screen page as shown in FIG. 12, clicking a data analysis button responds to a data analysis instruction, according to a movement difference between the contrast tracer agent and a tissue structure, a contrast tracer agent signal and a tissue signal can be separated through a signal processing method such as a subtraction and a filtering, to obtain a contrast tracer signal data. According to a first frame two-dimensional image corresponding to a first set of two-dimensional fundamental wave signal data, on a data analysis interface as shown in FIG. 13, a region of interest framing operation can be performed on the first frame two-dimensional image, when the region of interest is in an unselected state, a frame boundary is displayed as a dashed line, in a dashed line state, a size and a position of the region of interest can be edited, when the region of interest is determined, the frame boundary is displayed as a solid line, to obtain an ultrasound image corresponding to the region of interest.

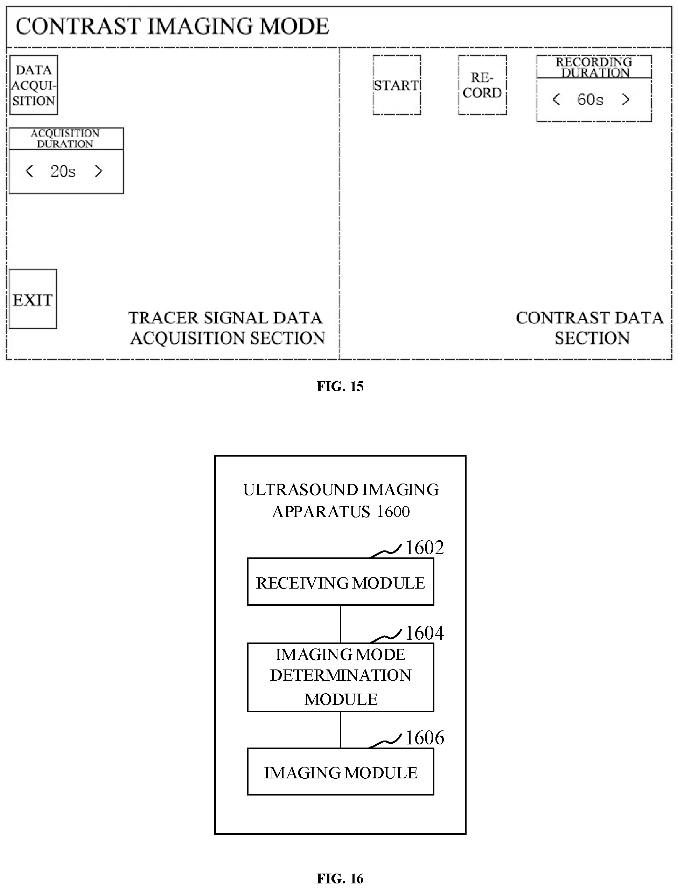

If the probe 602 is a medium-to-low frequency probe, under a lower medium-to-low frequency ultrasound wave transmission frequency, a resonance phenomenon amplitude of a contrast tracer agent is obvious, at this time the ultrasound imaging device 600 works in a contrast ultrasound imaging mode. FIG. 14 is a workflow diagram corresponding to the contrast ultrasound imaging mode, in the contrast ultrasound imaging mode, a tissue signal with a small movement amplitude can be effectively suppressed through a special transmission sequence, a simple filtering is performed on an acquired contrast agent signal, a contrast tracer agent signal separation effect is good, and an obtained ultrasound image has a high resolution. In the contrast imaging mode, as shown in FIG. 15, clicking a data acquisition button responds to a data acquisition instruction, a start button corresponding to a contrast data can be clicked at the same time to respond to a start instruction, and a data acquisition is performed simultaneously on the contrast data corresponding to a contrast agent signal and a tracer signal data corresponding to a contrast tracer agent signal.

Step 508, acquiring a motion parameter of each initial tracer signal, wherein, the motion parameter comprises a position parameter, a direction parameter and a velocity parameter; when the motion parameter meets a preset parameter condition, obtaining multiple target tracer signals.

Step 510, acquiring a first preprocessing parameter for controlling a number and an intensity of a brightness in an ultrasound image, and a second preprocessing parameter for compensating a movement of a target object; performing an analysis on each target tracer signal according to the first preprocessing parameter and the second preprocessing parameter to obtain an initial ultrasound image.

Step 512, acquiring an image processing parameter, wherein, the image processing parameter comprises an image resolution, an image contrast, an image filtering and an image enhancement; performing an image processing on the initial ultrasound image according to the image processing parameter to obtain a target ultrasound image.

In this embodiment, by acquiring an ultrasound echo signal of a target region, the ultrasound echo signal is an echo signal reflected after transmitting an ultrasound wave signal of a target transmission frequency to the target region; in the target region, a tracer substance is preset, according to a magnitude relationship between the target transmission frequency and a preset resonance frequency range of the tracer substance, a target imaging mode corresponding to the target region is determined; within a predetermined time period, according to the target imaging mode, performing signal acquisition on the ultrasound echo signal to obtain multiple target tracer signals, through the target imaging mode, performing localizing and tracking on each of the target tracer signals to obtain a target ultrasound image. The method determines a working mode during an ultrasound imaging process according to the target transmission frequency corresponding to the ultrasound wave signal, and can obtain a high-resolution ultrasound image at any target transmission frequency, based on the tracer substance, for a target region that is very small or a key region in a concealed position, for example, microvascular network ultrasound imaging, an accuracy and a precision of an ultrasound image can also be improved. And, if the method is applied to a field of medical detection, under a contrast imaging mode, a contrast agent signal and a tracer signal can be acquired simultaneously, according to the contrast agent signal, a detection analysis of a conventional contrast mode can be achieved, according to the tracer signal, a detection analysis of a super-resolution contrast imaging mode can be achieved, which can reduce a detection cost while improving a detection efficiency.

It should be understood that, although each step in a flowchart involved in each embodiment described above is displayed sequentially according to an indication of an arrow, these steps are not necessarily executed sequentially in an order indicated by the arrow. Unless explicitly stated herein, an execution of these steps is not strictly limited in order, and these steps can be executed in other orders. Moreover, at least a part of the steps in the flowchart involved in each embodiment described above can comprise multiple steps or multiple stages, these steps or stages are not necessarily executed and completed at a same moment, but can be executed at different moments, an execution order of these steps or stages is also not necessarily sequential, but can be executed in turn or alternately with other steps or at least a part of a step or a stage in other steps.

Based on a same inventive concept, an embodiment of the present application also provides an ultrasound imaging apparatus for implementing the above-involved ultrasound imaging method. An implementation solution for solving a problem provided by the apparatus is similar to an implementation solution recorded in the above method, therefore a specific limitation in one or more embodiments of the ultrasound imaging apparatus provided below can refer to a limitation on the ultrasound imaging method above, and will not be repeated here.

In an exemplary embodiment, as shown in FIG. 16, an ultrasound imaging apparatus 1600 is provided, comprising: a receiving module 1602, an imaging mode determination module 1604 and an imaging module 1606, wherein:

the receiving module 1602, configured to acquire an ultrasound echo signal of a target region, the ultrasound echo signal is an echo signal reflected after transmitting an ultrasound wave signal of a target transmission frequency to the target region;

the imaging mode determination module 1604, configured to provide a preset tracer substance in the target region, and determine a target imaging mode corresponding to the target region according to a magnitude relationship between the target transmission frequency and a preset resonance frequency range of the tracer substance;

the imaging module 1606, configured to perform signal acquisition on the ultrasound echo signal according to the target imaging mode within a predetermined time period to obtain multiple target tracer signals, and perform localizing and tracking on each target tracer signal through the target imaging mode to obtain a target ultrasound image.

In an embodiment, a target imaging mode comprises a two-dimensional fundamental wave imaging mode and a contrast imaging mode, the imaging mode determination module 1604 is also configured to, when a target transmission frequency is not within a preset resonance frequency range, determine the target imaging mode as the two-dimensional fundamental wave imaging mode, wherein, the two-dimensional fundamental wave imaging mode performs ultrasound imaging by acquiring a two-dimensional fundamental wave signal in an ultrasound echo signal; when the target transmission frequency is within the preset resonance frequency range, determine the target imaging mode as the contrast imaging mode, wherein, the contrast imaging mode performs ultrasound imaging by acquiring a contrast agent signal in the ultrasound echo signal.

In an embodiment, a two-dimensional fundamental wave signal comprises a tissue signal and a first tracer signal, the imaging module 1606 is also configured to, under a two-dimensional fundamental wave imaging mode, according to the two-dimensional fundamental wave imaging mode, perform signal acquisition on an ultrasound echo signal to obtain the two-dimensional fundamental wave signal; perform a spatiotemporal filtering and a frequency filtering on the two-dimensional fundamental wave signal respectively, to filter out the tissue signal, to obtain the first tracer signal.

In an embodiment, the imaging module 1606 is also configured to, within a predetermined time period, according to a movement of a tracer substance in a target region, obtain a number of initial tracer signals; acquire a motion parameter of each initial tracer signal, wherein, the motion parameter comprises a position parameter, a direction parameter and a velocity parameter; when the motion parameter meets a preset parameter condition, obtain multiple target tracer signals.

In an embodiment, the apparatus is also configured to acquire a first ultrasound echo signal of a target object, the first ultrasound echo signal is an echo signal reflected after transmitting an ultrasound wave signal to the target object; according to the first ultrasound echo signal, obtain a first ultrasound image, when a region of interest exists in the first ultrasound image, take the region of interest as the target region.

In an embodiment, the imaging module 1606 is also configured to acquire a first preprocessing parameter for controlling a number and an intensity of a brightness in an ultrasound image, and a second preprocessing parameter for compensating a movement of a target object; according to the first preprocessing parameter and the second preprocessing parameter, perform localizing and tracking on each target tracer signal to obtain an initial ultrasound image; acquire an image processing parameter, wherein, the image processing parameter comprises an image resolution, an image contrast, an image filtering and an image enhancement; according to the image processing parameter, perform an image processing on the initial ultrasound image to obtain the target ultrasound image.

Each module in the above ultrasound imaging apparatus can be implemented in whole or in part by a software, a hardware and a combination thereof. The above each module can be embedded in or independent of a processor in a computer device in a hardware form, or can be stored in a memory in the computer device in a software form, so that the processor can call and execute an operation corresponding to the above each module.

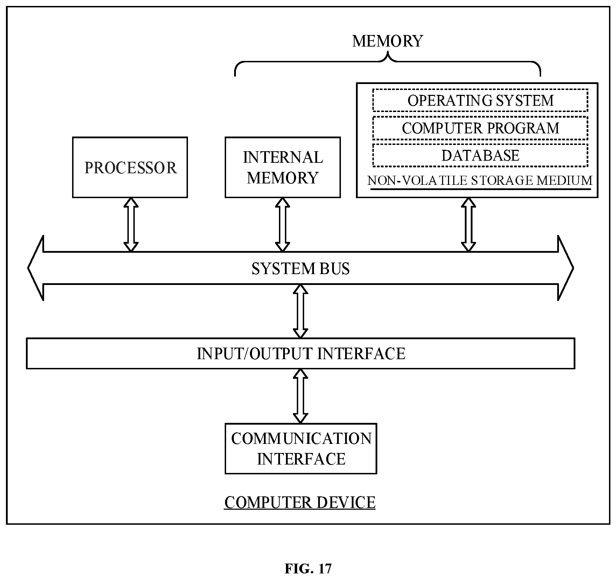

In an exemplary embodiment, a computer device is provided, the computer device can be a server, an internal structure diagram of the computer device can be as shown in FIG. 17. The computer device comprises a processor, a memory, an input/output interface (Input/Output, I/O for short) and a communication interface. Wherein, the processor, the memory and the input/output interface are connected through a system bus, the communication interface is connected to the system bus through the input/output interface. Wherein, the processor of the computer device is used to provide a computing and a control capability. The memory of the computer device comprises a non-volatile storage medium and an internal memory. The non-volatile storage medium stores an operating system, a computer program and a database. The internal memory provides an environment for an operation of the operating system and the computer program in the non-volatile storage medium. The database of the computer device is used to store an ultrasound imaging data. The input/output interface of the computer device is used for an exchange of an information between the processor and an external device. The communication interface of the computer device is used to communicate with an external terminal through a network connection. The computer program, when executed by the processor, implements an ultrasound imaging method.

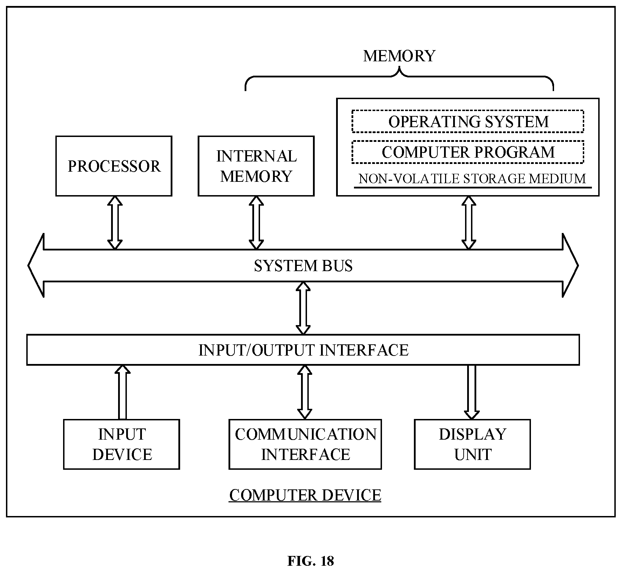

In an exemplary embodiment, a computer device is provided, the computer device can be a terminal, an internal structure diagram of the computer device can be as shown in FIG. 18. The computer device comprises a processor, a memory, an input/output interface, a communication interface, a display unit and an input device. Wherein, the processor, the memory and the input/output interface are connected through a system bus, the communication interface, the display unit and the input device are connected to the system bus through the input/output interface. Wherein, the processor of the computer device is used to provide a computing and a control capability. The memory of the computer device comprises a non-volatile storage medium and an internal memory. The non-volatile storage medium stores an operating system and a computer program. The internal memory provides an environment for an operation of the operating system and the computer program in the non-volatile storage medium. The input/output interface of the computer device is used for an exchange of an information between the processor and an external device. The communication interface of the computer device is used for a wired or a wireless communication with an external terminal, a wireless mode can be implemented through a WIFI, a mobile cellular network, an NFC (Near Field Communication) or other technology. The computer program, when executed by the processor, implements an ultrasound imaging method. The display unit of the computer device is used to form a visually visible picture, can be a display screen, a projection device or a virtual reality imaging device. The display screen can be a liquid crystal display screen or an electronic ink display screen, the input device of the computer device can be a touch layer covering the display screen, or can be a key, a trackball or a touchpad provided on a housing of the computer device, or can be an external keyboard, a touchpad or a mouse, etc.

A person skilled in the art can understand that the foregoing structure is merely a block diagram of a part of a structure related to a solution of the present application, and does not constitute a limitation on a computer device to which the solution of the present application is applied, a specific computer device can comprise more or fewer components than shown in a figure, or combine certain components, or have a different component arrangement.

In an embodiment, a computer device is provided, comprising a memory and a processor, the memory stores a computer program, the processor, when executing the computer program, implements the steps of each method embodiment above.

In an embodiment, a computer-readable storage medium is provided, on which a computer program is stored, the computer program, when executed by a processor, implements the steps of each method embodiment above.

In an embodiment, a computer product is provided, comprising a computer program, the computer program, when executed by a processor, implements the steps of each method embodiment above.

It needs to be explained that a user information (including but not limited to a user device information, a user personal information, etc.) and a data (including but not limited to a data for an analysis, a stored data, a displayed data, etc.) involved in the present application are all an information and a data authorized by a user or fully authorized by each party, and a collection, a use and a processing of a relevant data need to comply with a relevant regulation.

A person of ordinary skill in the art can understand that all or a part of a process in a method of the above embodiment can be completed by a computer program instructing a relevant hardware, the computer program can be stored in a non-volatile computer-readable storage medium, the computer program, when executed, can comprise the process of the embodiment of each method above. Wherein, any reference to a memory, a database or other medium used in each embodiment provided by the present application can comprise at least one of a non-volatile and a volatile memory. The non-volatile memory can comprise a Read-Only Memory (ROM), a magnetic tape, a floppy disk, a flash memory, an optical memory, a high-density embedded non-volatile memory, a Resistive Random Access Memory (ReRAM), a Magnetoresistive Random Access Memory (MRAM), a Ferroelectric Random Access Memory (FRAM), a Phase Change Memory (PCM), a graphene memory, etc. The volatile memory can comprise a Random Access Memory (RAM) or an external cache memory, etc. As an illustration and not a limitation, the RAM can be in a variety of forms, such as a Static Random Access Memory (SRAM) or a Dynamic Random Access Memory (DRAM), etc. A database involved in each embodiment provided by the present application can comprise at least one of a relational database and a non-relational database. The non-relational database can comprise a blockchain-based distributed database, etc., but is not limited to this. A processor involved in each embodiment provided by the present application can be a general-purpose processor, a central processing unit, a graphics processing unit, a digital signal processor, a programmable logic device, a quantum computing-based data processing logic device, etc., but is not limited to this.

Each technical feature of the above embodiments can be arbitrarily combined, to make a description concise, not all possible combinations of each technical feature in the above embodiments are described, however, as long as a combination of these technical features does not have a contradiction, it should be considered as a scope recorded in this specification.

The above embodiments only express several implementation modes of the present application, their descriptions are relatively specific and detailed, but cannot be understood as a limitation on a patent scope of the present application for this reason. It should be pointed out that, for a person of ordinary skill in the art, without departing from a concept of the present application, several variations and improvements can also be made, these all belong to a protection scope of the present application. Therefore, the protection scope of the present application should be subject to the appended claims.

## Claims

1. An ultrasound imaging method, **characterized in that** it comprises:
acquiring an ultrasound echo signal of a target region, the ultrasound echo signal being an echo signal reflected after transmitting an ultrasound wave signal of a target transmission frequency to the target region;
providing a preset tracer substance in the target region, and determining a target imaging mode corresponding to the target region according to a magnitude relationship between the target transmission frequency and a preset resonance frequency range of the tracer substance;
within a predetermined time period, performing signal acquisition on the ultrasound echo signal according to the target imaging mode to obtain multiple target tracer signals, and performing localizing and tracking on each of the target tracer signals through the target imaging mode to obtain a target ultrasound image.

2. The method of claim 1, **characterized in that**, the target imaging mode comprises a two-dimensional fundamental wave imaging mode and a contrast imaging mode, and determining the target imaging mode corresponding to the target region according to the magnitude relationship between the target transmission frequency and the preset resonance frequency range of the tracer substance, comprises:
when the target transmission frequency is not within the preset resonance frequency range, determining the target imaging mode as the two-dimensional fundamental wave imaging mode, wherein the two-dimensional fundamental wave imaging mode performs ultrasound imaging by acquiring a two-dimensional fundamental wave signal in the ultrasound echo signal;
when the target transmission frequency is within the preset resonance frequency range, determining the target imaging mode as the contrast imaging mode, wherein the contrast imaging mode performs ultrasound imaging by acquiring a contrast agent signal in the ultrasound echo signal.

3. The method of claim 2, **characterized in that**, the two-dimensional fundamental wave signal comprises a tissue signal and a first tracer signal, and performing signal acquisition on the ultrasound echo signal according to the target imaging mode to obtain a target tracer signal, comprises:
under the two-dimensional fundamental wave imaging mode, performing signal acquisition on the ultrasound echo signal according to the two-dimensional fundamental wave imaging mode to obtain the two-dimensional fundamental wave signal;
performing a spatiotemporal filtering and a frequency filtering on the two-dimensional fundamental wave signal respectively to filter out the tissue signal and obtain the first tracer signal.

4. The method of claim 1, **characterized in that**, performing signal acquisition on the ultrasound echo signal according to the target imaging mode within a predetermined time period to obtain multiple target tracer signals, comprises:
within the predetermined time period, obtaining a plurality of initial tracer signals according to a movement of the tracer substance in the target region;
acquiring a motion parameter of each of the initial tracer signals, wherein the motion parameter comprises a position parameter, a direction parameter and a velocity parameter;
when the motion parameter meets a preset parameter condition, obtaining multiple of the target tracer signals.

5. The method of claim 1, **characterized in that**, before acquiring an ultrasound echo signal of a target region, the method comprises:
acquiring a first ultrasound echo signal of a target object, the first ultrasound echo signal being an echo signal reflected after transmitting an ultrasound wave signal to the target object;
obtaining a first ultrasound image according to the first ultrasound echo signal, and when a region of interest exists in the first ultrasound image, treating the region of interest as the target region.

6. The method of claim 5, **characterized in that**, processing the target tracer signal through the target imaging mode to obtain a target ultrasound image comprises:
acquiring a first preprocessing parameter for controlling a number and an intensity of a brightness in an ultrasound image, and a second preprocessing parameter for compensating a movement of the target object;
performing localizing and tracking on each of the target tracer signals according to the first preprocessing parameter and the second preprocessing parameter to obtain an initial ultrasound image;
acquiring an image processing parameter, wherein, the image processing parameter comprises an image resolution, an image contrast, an image filtering and an image enhancement;
performing an image processing on the initial ultrasound image according to the image processing parameter to obtain the target ultrasound image.

7. An ultrasound imaging apparatus, **characterized in that**, the apparatus comprises:
a receiving module, configured to acquire an ultrasound echo signal of a target region, the ultrasound echo signal being an echo signal reflected after transmitting an ultrasound wave signal of a target transmission frequency to the target region;
an imaging mode determination module, configured to provide a preset tracer substance in the target region, and determine a target imaging mode corresponding to the target region according to a magnitude relationship between the target transmission frequency and a preset resonance frequency range of the tracer substance;
an imaging module, configured to, perform signal acquisition on the ultrasound echo signal according to the target imaging mode within a predetermined time period to obtain multiple target tracer signals, and perform localizing and tracking on each of the target tracer signals through the target imaging mode to obtain a target ultrasound image.

8. A computer device, comprising a memory and a processor, the memory storing a computer program, **characterized in that**, the processor implements the steps of the method of any one of claims 1 to 6 when executing the computer program.

9. A computer-readable storage medium, on which a computer program is stored, **characterized in that**, the computer program implements the steps of the method of any one of claims 1 to 6 when executed by a processor.

10. A computer product, comprising a computer program, **characterized in that**, the computer program implements the steps of the method of any one of claims 1 to 6 when executed by a processor.
